# EUROPEAN PATENT SPECIFICATION

(11) **EP 3 203 239 B1**
(45) Date of publication and mention of the grant of the patent: **26.09.2018**
(21) Application number: 17154500.7
(22) Date of filing: 03.02.2017
(51) Int. Cl.: G01N 33/68

(54) **METHOD FOR DETERMINING ATRIAL FIBRILLATION**
VERFAHREN ZUR BESTIMMUNG VON VORHOFFLIMMERN
PROCÉDÉ DE DÉTERMINATION DE LA FIBRILLATION AURICULAIRE

(30) Priority: 03.02.2016 JP 2016019069
(43) Date of publication of application: 09.08.2017
(73) Proprietor: Matsumori, Akira, Minoh-shi, Osaka 562-0005 (JP)
(72) Inventor: Matsumori, Akira, Minoh-shi, Osaka 562-0005 (JP)
(74) Representative: Paemen, Liesbet R.J.

(56) References cited:
- US-A1- 2008 033 258
- US-A1- 2011 159 487
- US-A1- 2014 170 771
- GRISENDI SILVIA ET AL: "Nucleophosmin and cancer", NATURE REVIEWS. CANCER, NATUR PUBLISHING GROUP, LONDON, GB, vol. 6, no. 7, 1 July 2006 (2006-07-01), pages 493-505, XP002559721, ISSN: 1474-175X, DOI: 10.1038/NRC1885

### SEQUENCE LISTING

<110> Matsumori, Akira
<120> Determination method of atrial fibrillation
<130> MTS-001-EP
<150> JP2016-019069
   <151> 2016-02-03
<160> 4
<170> PatentIn version 3.5
<210> 1
   <211> 294
   <212> PRT
   <213> Homo sapiens
<400> 1
   Met Glu Asp Ser Met Asp Met Asp Met Ser Pro Leu Arg Pro Gln Asn
   1               5                   10                  15
   Tyr Leu Phe Gly Cys Glu Leu Lys Ala Asp Lys Asp Tyr His Phe Lys
               20                  25                  30
   Val Asp Asn Asp Glu Asn Glu His Gln Leu Ser Leu Arg Thr Val Ser
           35                  40                  45
   Leu Gly Ala Gly Ala Lys Asp Glu Leu His Ile Val Glu Ala Glu Ala
       50                  55                  60
   Met Asn Tyr Glu Gly Ser Pro Ile Lys Val Thr Leu Ala Thr Leu Lys
   65                  70                  75                  80
   Met Ser Val Gln Pro Thr Val Ser Leu Gly Gly Phe Glu Ile Thr Pro
                   85                  90                  95
   Pro Val Val Leu Arg Leu Lys Cys Gly Ser Gly Pro Val His Ile Ser
               100                 105                 110
   Gly Gln His Leu Val Ala Val Glu Glu Asp Ala Glu Ser Glu Asp Glu
           115                 120                 125
   Glu Glu Glu Asp Val Lys Leu Leu Ser Ile Ser Gly Lys Arg Ser Ala
       130                 135                 140
   Pro Gly Gly Gly Ser Lys Val Pro Gln Lys Lys Val Lys Leu Ala Ala
   145                 150                 155                 160
   Asp Glu Asp Asp Asp Asp Asp Asp Glu Glu Asp Asp Asp Glu Asp Asp
                   165                 170                 175
   Asp Asp Asp Asp Phe Asp Asp Glu Glu Ala Glu Glu Lys Ala Pro Val
               180                 185                 190
   Lys Lys Ser Ile Arg Asp Thr Pro Ala Lys Asn Ala Gln Lys Ser Asn
           195                 200                 205
   Gln Asn Gly Lys Asp Ser Lys Pro Ser Ser Thr Pro Arg Ser Lys Gly
       210                 215                 220
   Gln Glu Ser Phe Lys Lys Gln Glu Lys Thr Pro Lys Thr Pro Lys Gly
   225                 230                 235                 240
   Pro Ser Ser Val Glu Asp Ile Lys Ala Lys Met Gln Ala Ser Ile Glu
                   245                 250                 255
   Lys Gly Gly Ser Leu Pro Lys Val Glu Ala Lys Phe Ile Asn Tyr Val
               260                 265                 270
   Lys Asn Cys Phe Arg Met Thr Asp Gln Glu Ala Ile Gln Asp Leu Trp
           275                 280                 285
   Gln Trp Arg Lys Ser Leu
       290
<210> 2
   <211> 298
   <212> PRT
   <213> Homo sapiens
<400> 2
<210> 3
   <211> 1296
   <212> RNA
   <213> Homo sapiens
<400> 3
<210> 4
   <211> 1092
   <212> RNA
   <213> Homo sapiens
<400> 4
   gguuguucuc uggagcagcg uucuuuuauc uccguccgcc uucucuccua ccuaagugcg     60
   ugccgccacc cgauggaaga uucgauggac auggacauga gcccccugag gccccagaac    120
   uaucuuuucg guugugaacu aaaggccgac aaagauuauc acuuuaaggu ggauaaugau    180
   gaaaaugagc accaguuauc uuuaagaacg gucaguuuag gggcuggugc aaaggaugag    240
   uugcacauug uugaagcaga ggcaaugaau uacgaaggca guccaauuaa aguaacacug    300
   gcaacuuuga aaaugucugu acagccaacg guuucccuug ggggcuuuga aauaacacca    360
   ccaguggucu uaagguugaa gugugguuca gggccagugc auauuagugg acagcacuua    420
   guagcugugg aggaagaugc agagucagaa gaugaagagg aggaggaugu gaaacucuua    480
   aguauaucug gaaagcgguc ugccccugga ggugguagca agguuccaca gaaaaaagua    540
   aaacuugcug cugaugaaga ugaugacgau gaugaugaag aggaugauga ugaagaugau    600
   gaugaugaug auuuugauga ugaggaagcu gaagaaaaag cgccagugaa gaaaucuaua    660
   cgagauacuc cagccaaaaa ugcacaaaag ucaaaucaga auggaaaaga cucaaaacca    720
   ucaucaacac caagaucaaa aggacaagaa uccuucaaga aacaggaaaa aacuccuaaa    780
   acaccaaaag gaccuaguuc uguagaagac auuaaagcaa aaaugcaagc aaguauagaa    840
   aaaggugguu cucuucccaa aguggaagcc aaauucauca auuaugugaa gaauugcuuc    900
   cggaugacug accaagaggc uauucaagau cucuggcagu cccuggagaa agucucuuua    960
   agaaaauagu uuaaacaauu uguuaaaaaa uuuuccgucu uauuucauuu cuguaacagu   1020
   ugauaucugg cuguccuuuu uauaaugcag agugagaacu uucccuaccg uguuugauaa   1080
   auguugucca gg                                                       1092

## Description

### TECHNICAL FIELD

The present invention relates to a method for supporting a diagnosis of atrial fibrillation.

### BACKGROUND

As a biomarker of heart disease, BNP, NT-proBNP, cardiac troponin T, cardiac troponin I and the like are used. BNP and NT-proBNP are increased with heart failure (systolic dysfunction). BNP and NT-proBNP are known to correlate with the severity of heart failure (Hanna K. Gaggin, and James L. Januzzi Jr.; Biochimica et Biophysica Acta 1832 (2013) 2442-2450. "Biomarkers and diagnostics in heart failure"). Cardiac troponin T and cardiac troponin I are markers of cardiomyocyte necrosis. Cardiac troponin T and cardiac troponin I are reported to be useful for a diagnosis of acute myocardial infarction, but usually not to be increased with heart failure (Peacock WF 4th et al.; N Engl J Med. 2008 May 15; 358(20): 2117-26. "Cardiac troponin and outcome in acute heart failure."). BNP/NT-proBNP are increased with cardiac hypertrophy without heart failure (also called as cardiac hypertrophy due to hypertension, hypertrophic cardiomyopathy or dystelectasis) and arrhythmia such as atrial fibrillation (McKie PM, and Burnett JC Jr.; Mayo Clin Proc. 2005 Aug; 80(8): 1029-36. "B-type natriuretic peptide as a biomarker beyond heart failure: speculations and opportunities.").

### SUMMARY OF THE INVENTION

In atrial fibrillation, contribution of atrial contraction to ventricular filling is lost due to rapid and irregular atrial activation, and cardiac output is decreased. The decrease in the cardiac output results in hemodynamic compromise and becomes a heart failure exacerbating factor. Decreased intra-atrial blood flow velocity due to atrial fibrillation can cause thrombogenesis and is eventually accompanied by embolism such as cerebral infarction. Therefore, the course of treatment for atrial fibrillation includes antithrombotic treatment, in addition to regulation of heart rate, maintenance of sinus rhythm and relapse prevention. Early detection of atrial fibrillation is important, also in determining a timing to start antithrombotic treatment. A patient with atrial fibrillation may have subjective symptoms such as palpitation and chest symptoms or may be asymptomatic, thus it is necessary to detect atrial fibrillation in an early stage, based on other than subjective symptoms. However, BNP/NT-proBNP measured value in the blood currently reported to be increased in atrial fibrillation is also increased with heart failure and cardiac hypertrophy, thus it is not possible to specifically detect atrial fibrillation. Obtainment of information on atrial fibrillation, distinguished from heart failure and cardiac hypertrophy, is useful in the judgment for preventing and treating embolism. However, a marker that can clearly distinguish atrial fibrillation from other heart diseases is not currently known.

The present inventor has intensively studied, and found that the measured value of nucleophosmin in a biological sample of a patient with atrial fibrillation is significantly high as compared with the value of a healthy subject and other patients with heart disease, and completed the present invention.

The present invention has been completed based on the above knowledge, and comprises the following aspects.
Item 1. A method for supporting a diagnosis of atrial fibrillation comprising:
   (a) a step of obtaining a measured value of nucleophosmin in a biological sample collected from a test subject; and
   (b) a step of obtaining information on atrial fibrillation, based on the obtained measured value of nucleophosmin.
Item 2. The method according to item 1, wherein the biological sample is a blood sample.
Item 3. The method according to claim 1 or 2, wherein
   the measured value of nucleophosmin and a predetermined threshold are compared in the step (b), and
   when the measured value is the threshold or more, information showing that the test subject has atrial fibrillation is obtained.
Item 4. The method according to claim 1 or 2, wherein
   the measured value of nucleophosmin and a predetermined threshold are compared in the step (b), and
   when the measured value is less than the threshold, information showing that the test subject does not have atrial fibrillation is obtained.
Item 5. The method according to any one of claims 1 to 4, wherein the measured value of nucleophosmin is an expression level of a nucleophosmin gene or a measured value of a nucleophosmin protein.
Item 6. Use of a reagent for the method for supporting a diagnosis of atrial fibrillation according to any one of claims 1 to 5, wherein
   the reagent comprises an anti-nucleophosmin antibody.
Item 7. Use of a kit for the method for supporting a diagnosis of atrial fibrillation according to any one of claims 1 to 5, the kit comprising:
   a first anti-nucleophosmin antibody;
   a solid phase;
   a second anti-nucleophosmin antibody; and
   a labeling substance.

The present invention can provide a method for supporting a diagnosis of atrial fibrillation.

### BRIEF DESCRIPTION OF THE DRAWINGS

Fig. 1 is a schematic view showing an example of an inspection kit.
Fig. 2 is a schematic view showing an example of a diagnosis supporting device.
Fig. 3 is a block diagram showing a hardware configuration of a diagnosis supporting device.
Fig. 4 is a flow chart of a method for detecting a heart failure patient using a diagnosis supporting device.
Fig. 5 shows a scatter diagram of a nucleophosmin concentration in the serum of each group.
Fig. 6A shows an ROC curve of the measured value of nucleophosmin of an Af patient group and the measured value of nucleophosmin of a dilated cardiomyopathy patient group.
Fig. 6B shows an ROC curve of the measured value of nucleophosmin of an Af patient group and the measured value of nucleophosmin of a hypertrophic cardiomyopathy patient group.
Fig. 6C shows an ROC curve of the measured value of nucleophosmin of an Af patient group and the measured value of nucleophosmin of a hypertension patient group.
Fig. 6D shows an ROC curve of the measured value of nucleophosmin of an Af patient group and the measured value of nucleophosmin of an ischemic cardiomyopathy patient group.
Fig. 6E shows an ROC curve of the measured value of nucleophosmin of an Af patient group and the measured value of nucleophosmin of a healthy subject group.

### DETAILED DESCRIPTION OF THE PREFERRED EMBODIMENTS

### 1. Explanation of terms

First, the terms used in the present specification, claims and abstract will be explained.

In the present specification, the "atrial fibrillation" is a type of arrhythmia, and is a disease in which small irregular baseline fluctuation is found on an electrocardiogram. In the present disclosure, atrial fibrillation also includes asymptomatic atrial fibrillation, in addition to atrial fibrillation accompanying subjective symptoms such as palpitation. Atrial fibrillation may be any of initial, paroxysmal, persistent, long-lasting and permanent atrial fibrillation. Furthermore, the cause of atrial fibrillation is not also particularly limited. Examples of the cause of atrial fibrillation include valvular disease, metabolic syndrome, hypertension, ischemic heart disease, and the like.

In the present disclosure, the "individual" is not particularly limited, but the individual includes humans and mammals other than humans. Examples of mammals other than humans include cows, horses, sheep, goats, pigs, dogs, cats, rabbits, monkeys, and the like. The individual is preferably human. Age and sex of the individual do not matter.

The "test subject" may be an individual having a history of atrial fibrillation and other heart diseases or an individual having no history of atrial fibrillation and other heart diseases. The test subject may be a subject having subjective symptoms such as palpitation, pain or discomfort in the chest, stagger or general malaise, or a subject having no symptoms. The test subject also includes a specimen suspected of having atrial fibrillation, in accordance with known diagnosis criteria such as physiological tests such as medical interview, electrocardiogram, and mechanocardiography.

In the present disclosure, the "biological sample" includes biologically derived cells, tissues, urine and blood samples, and the like. The biological sample is preferably the "blood sample".

The "blood sample" refers to blood (whole blood) collected from a test subject, or serum or plasma prepared from the blood. The blood sample is more preferably serum or plasma, and further preferably serum. The kind of the anticoagulant used when collecting plasma is not particularly limited. A blood sample of a test subject to be used in the measurement and a blood sample to determine a predetermined threshold may be the same or different kind, and are preferably the same kind. When plasma is used as the blood sample, plasma for determining a predetermined threshold is preferably prepared from a blood collected using the same anticoagulant as that used in the plasma of the test subject. The blood sample may be fresh or stored blood. When the blood sample is stored, it can be stored in a room temperature environment, a refrigerated environment or a freezing environment, and frozen storage is preferred.

Nucleophosmin (NPM/B23) is a protein that shuttles between nucleus and cytoplasm. Herein, it is sometimes called as "nucleophosmin protein" when particularly referring to the protein of nucleophosmin, and it is sometimes called as "nucleophosmin gene" when particularly referring to the gene encoding nucleophosmin.

The "measured value of nucleophosmin" includes a "measured value of a nucleophosmin protein" and an "expression level of a nucleophosmin gene" in a biological sample.

The "measured value of a nucleophosmin protein" refers to a value reflecting the amount or concentration of the nucleophosmin protein. When the measured value is written by "amount", it may be written by mol or mass, and is preferably written by mass. When the value is written by "concentration", it may be written by mol concentration or a mass ratio per constant volume (mass/volume) of a biological sample, and is preferably written by mass/volume. As the value reflecting the amount or concentration, intensity of signal such as fluorescence and luminescence may be used, in addition to the those described above.

The "expression level of a nucleophosmin gene" refers to a value reflecting an expression level of nucleophosmin mRNA. The expression level may be represented by a copy number (absolute amount) of nucleophosmin mRNA present in a constant amount of biological sample, or may be a value reflecting an expression level relative to the expression level of a house keeping gene such as β2-microglobulin mRNA, GAPDH mRNA and β-actin mRNA. The expression level may be expressed by intensity of signal such as fluorescence and luminescence.

The "predetermined threshold" refers to a threshold of the measured value of nucleophosmin. The threshold can be determined based on the measured value of nucleophosmin in a biological sample of an individual who has not developed atrial fibrillation and/or the measured value of nucleophosmin in a biological sample of an individual who has developed atrial fibrillation.

For example, the measured values of nucleophosmin measured using biological samples of a plurality of individuals who have developed atrial fibrillation and the measured values of nucleophosmin measured using biological samples of a plurality of individuals who have not developed atrial fibrillation are obtained. The value capable of most accurately classifying into positive or negative based on a plurality of these values can be defined as a "threshold". The "value capable of most accurately classifying" can be appropriately set, based on indexes such as sensitivity, specificity, positive predictive value, and negative predictive value, according to the purpose of inspection.

For example, as one embodiment, among the measured values of nucleophosmin in each biological sample obtained from a plurality of individuals who have developed atrial fibrillation, the lowest measured value may be defined as a threshold. For example, when reducing false positive as much as possible, like a screening inspection, said threshold can be suitably used.

As another embodiment, when determining a threshold based on the measured values of nucleophosmin in a biological sample of an individual who has not developed atrial fibrillation, among the measured values of nucleophosmin in biological samples obtained from a plurality of individuals who have not developed atrial fibrillation, the highest measured value can be determined as a threshold. For example, when reducing false negative as much as possible, said threshold can be suitably used.

As another embodiment, a threshold can be also defined as the measured value of nucleophosmin in a biological sample of an individual who has not developed atrial fibrillation as it is, or an average, a median or a mode of a plurality of the measured values of nucleophosmin of an individual who has not developed atrial fibrillation.

A threshold can be also determined based on the measured values of nucleophosmin in a plurality of biological samples of an individual who has not developed atrial fibrillation.

In this case, a threshold can be defined as [the average of the measured values of nucleophosmin] in a plurality of biological samples, preferably [a value obtained by subtracting "a value obtained by multiplying a standard deviation of the measured values of nucleophosmin in a plurality of biological samples by one" from "the average"], or more preferably [a value obtained by subtracting "a value obtained by multiplying the standard deviation by two" from "the average"].

As a threshold, a past measured value (that may be one value, or may be an average, a median or a mode of a plurality of values) of nucleophosmin obtained in the same test subject before the test subject has atrial fibrillation can be also used.

When a threshold is determined based on the measured value of nucleophosmin in a biological sample of an individual who has not developed atrial fibrillation and the measured value of nucleophosmin in a biological sample of an individual who has developed atrial fibrillation, the average of the measured value of nucleophosmin in a biological sample of a single individual who has not developed atrial fibrillation and the measured value of nucleophosmin in a biological sample of a single individual who has developed atrial fibrillation can be used as a reference. A value obtained by further averaging "an average of the measured values of nucleophosmin in a plurality of biological samples of an individual who has not developed atrial fibrillation" and "an average of the measured values of nucleophosmin in a plurality of biological samples of an individual who has developed atrial fibrillation" can be also used as a threshold. As another embodiment, individuals who have not developed atrial fibrillation and individuals who have developed atrial fibrillation are defined as one group, and a median of the measured values of nucleophosmin in biological samples of this one group can be also used as a threshold.

As still another embodiment, in the method of determining a threshold, in place of the measured value of nucleophosmin in a biological sample of an individual who has not developed atrial fibrillation, the measured value of nucleophosmin in a biological sample of a healthy individual may be used.

These thresholds may be determined when obtaining the measured value of nucleophosmin in a biological sample of a test subject, or may be previously determined.

The "healthy individual" is not particularly limited. Preferably, the "healthy individual" is a human or a mammal other than humans as described in the section of the "individual". The "healthy individual" refers to an individual that does not show abnormal data in a biochemical test, blood test, urine test, serum test, physiological test, and the like. Age and sex of the healthy individual are not particularly limited.

The "individual who has not developed atrial fibrillation" is not particularly limited. Preferably, the "individual who has not developed atrial fibrillation" is a human or a mammal other than humans as described in the section of the "individual". The "individual who has not developed atrial fibrillation" is an individual who cannot be diagnosed to develop atrial fibrillation, in accordance with known diagnosis criteria.

"A plurality of biological samples" is 2 or more, preferably 5 or more, and more preferably 10 or more biological samples. These may be biological samples collected from different individuals or may be a plurality of biological samples of the same individual collected at different timings.

"A plurality of values" is 2 or more, preferably 5 or more, and more preferably 10 or more measured values of nucleophosmin.

"A plurality of individuals" is 2 or more, preferably 5 or more, and more preferably 10 or more individuals.

The species, age, sex and the like of the test subject are not necessarily the same as those of the individual to obtain the measured value of nucleophosmin for determining a threshold, but they are preferably the same species. The individual is preferably the same generation and/or the same sex as the test subject.

The "anti-nucleophosmin antibody" is not limited as long as it specifically binds to a nucleophosmin protein, and any of a polyclonal antibody, a monoclonal antibody and fragments thereof (for example, Fab, F(ab)₂, and the like) that are obtained by immunizing an animal other than humans with a nucleophosmin protein or a part thereof as an antigen can be used. Class and subclass of immunoglobulin are not particularly limited. The class and subclass of immunoglobulin may be a chimeric antibody, scFv, or the like.

The nucleophosmin protein to be an antigen used for preparing an anti-nucleophosmin antibody is preferably a nucleophosmin protein derived from human (for example, GenBank: AAA36380.1 [SEQ ID NO: 1], GenBank: AAW67757.1 [SEQ ID NO: 2]), mouse (for example, NCBI Reference Sequence: NP_032748.1, NP_001239189.1, NP_001239190.1), rat (for example, NCBI Reference Sequence: NP_037124.1) or the like, and more preferably a human nucleophosmin protein of SEQ ID NO: 1 or SEQ ID NO: 2. The nucleophosmin protein to be used as an antigen may be extracted from mammalian cells according to a known method, and may be a recombinant protein obtained according to a recombinant genetic engineering technique. When a part of the nucleophosmin protein is used as an antigen, a fragment obtained by digesting the nucleophosmin protein with an enzyme or the like may be used, and a peptide having the same sequence as the amino acid sequence of a part of the nucleophosmin protein may be used as an antigen. The peptide can be synthesized by a known method.

Further, as the anti-nucleophosmin antibody, for example, a commercial product such as an anti-nucleophosmin antibody [FC82291] (ab10530) reacted with a human nucleophosmin protein from Abcam plc. (UK, Cambridge) can be also used.

In the present disclosure, the "nucleophosmin mRNA" includes human (for example, GenBank: M23613.1 [SEQ ID NO: 3], GenBank: AY740639.1 [SEQ ID NO: 4]), mouse (for example, GenBank: GU214027.1), rat (for example, NCBI Reference Sequence: NM_012992.4), and the like. The nucleophosmin mRNA is more preferably human nucleophosmin mRNA of SEQ ID NO: 3 or SEQ ID NO: 4.

In the present disclosure, either total RNA or mRNA extracted from a biological sample may be used. The biological sample used in extraction of total RNA and mRNA is preferably subjected to RNA extraction shortly after being collected from an individual, or frozen (preferably rapidly cooled in an atmosphere of -80°C or less) shortly after being collected from an individual, and stored at -80°C or less until RNA extraction.

The method of extracting total RNA and mRNA from a biological sample is not particularly limited, and a known extraction method can be used.

### 2. Method for determining atrial fibrillation

In the methods provided herein, the measured value of nucleophosmin in a biological sample is obtained, and whether or not a test subject has atrial fibrillation is determined using the measured value of nucleophosmin.

### (i) Step of obtaining measured value of nucleophosmin

In the present step, first, a measured value of nucleophosmin in a biological sample collected from a test subject is obtained.

A method for obtaining the measured value of nucleophosmin in a biological sample is not particularly limited as long as the measured value of nucleophosmin contained in the biological sample can be obtained.

### (i-1) Obtainment of measured value of nucleophosmin protein

When the measured value of nucleophosmin is a measured value of a nucleophosmin protein, it is possible in the present step to use an antibody which can bind specifically to the nucleophosmin protein, namely, an anti-nucleophosmin antibody, in order to obtain the measured value. The "4. An inspection reagent for atrial fibrillation containing an anti-nucleophosmin antibody" or "5. An inspection kit for atrial fibrillation" described below may be used.

The order of mixing a biological sample and an anti-nucleophosmin antibody is not particularly limited, and these may be mixed substantially at the same time, or may be successively mixed.

In the methods provided herein, a complex of an anti-nucleophosmin antibody and a nucleophosmin protein in a biological sample is formed in advance and the complex is then immobilized on a solid phase, or an anti-nucleophosmin antibody is previously immobilized on a solid phase, and the immobilized anti-nucleophosmin antibody and a nucleophosmin protein in a biological sample can form a complex. More preferably, it is an embodiment that the complex is formed in advance, and then the complex is immobilized on a solid phase. Moreover, the complex immobilized on a solid phase or the complex formed on a solid phase is detected by a method known in the art, whereby the amount or concentration of a nucleophosmin protein contained in a biological sample can be measured.

When a complex of an anti-nucleophosmin antibody and a nucleophosmin protein in a biological sample is formed in advance and the complex is then immobilized on a solid phase, an anti-nucleophosmin antibody modified with biotin or the like is brought into contact with a nucleophosmin protein in a biological sample to form a complex. Separately, avidins are previously bound to the solid phase, whereby the complex can be immobilized on a solid phase via binding between biotin and avidins.

When an anti-nucleophosmin antibody is previously immobilized on a solid phase, the method of immobilization of an anti-nucleophosmin antibody to a solid phase is not particularly limited. For example, an anti-nucleophosmin antibody and a solid phase may be directly bound, or an anti-nucleophosmin antibody and a solid phase may be indirectly bound via another substance. Examples of direct binding include physical adsorption and the like. Examples of indirect binding include binding via a combination of biotin with avidin or streptavidin (hereinafter, also referred to as "avidins"). In this case, an anti-nucleophosmin antibody is previously modified with biotin, and avidins are previously bound to a solid phase, whereby an anti-nucleophosmin antibody and a solid phase can be indirectly bound via the binding between biotin and avidins. In the methods envisaged herein, the binding between an anti-nucleophosmin antibody and a solid phase is preferably an indirect binding via biotin and avidins.

The material of the solid phase is not particularly limited. For example, the material of the solid phase can be selected from organic polymer compounds, inorganic compounds, biopolymers, and the like. Examples of the organic polymer compounds include latex, polystyrene, polypropylene, and the like. Examples of the inorganic compounds include magnetic bodies (iron oxide, chromium oxide and ferrite, and the like), silica, alumina, glass, and the like. Examples of the biopolymers include insoluble agarose, insoluble dextran, gelatin, cellulose, and the like. Two or more kinds of these materials may be used in combination. A shape of the solid phase is not particularly limited, and examples include a particle, a membrane, a microplate, a microtube, a test tube, and the like. Among them, a particle is preferred, and a magnetic particle is particularly preferred.

In the present step, after the formation of the complex, preferably after the complex formation and before detection of a labeling substance, B/F separation that removes unreacted free components not forming the complex may be carried out. The unreacted free component refers to a component that does not constituting a complex. Examples of the unreacted free component include an anti-nucleophosmin antibody that does not bind to a nucleophosmin protein and the like. Means of B/F separation is not particularly limited. When the solid phase is a particle, only a solid phase capturing the complex is collected by centrifugation, whereby B/F separation can be carried out. When the solid phase is a container such as a microplate or microtube, B/F separation can be carried out by removing a liquid containing unreacted free components. When the solid phase is a magnetic particle, B/F separation can be carried out by sucking and removing a liquid containing unreacted free components by a nozzle while magnetic particles are magnetically restricted with a magnet. The method is preferred from the viewpoint of automation. After removing unreacted free components, the solid phase capturing the complex may be washed with a suitable aqueous medium such as PBS.

In the present step, detection of the complex can be performed by using an anti-nucleophosmin antibody labeled with a labeling substance, or can be performed by using an unlabeled anti-nucleophosmin antibody and an anti-immunoglobulin antibody or the like labeled with a labeling substance capable of binding to the unlabeled anti-nucleophosmin antibody, and it is preferred to use a labeled anti-nucleophosmin antibody. An epitope in a nucleophosmin protein of the labeled anti-nucleophosmin antibody and an epitope in a nucleophosmin protein of the anti-nucleophosmin antibody bound to the solid phase are preferably different.

The labeling substance used for a labeled anti-nucleophosmin antibody or labeled anti-immunoglobulin antibody is not particularly limited, as long as a detectable signal is generated. For example, the labeling substance may be a substance generating a signal in itself (hereinafter, also referred to as "signal-generating substance"), or may be a substance generating a signal by catalyzing a reaction of other substance. Examples of the signal-generating substance include fluorescent substances, radioisotopes, and the like. Examples of the substance generating a signal detectable by catalyzing a reaction of other substance include enzymes and the like. Examples of the enzymes include alkaline phosphatase, peroxidase, β-galactosidase, luciferase, and the like. Examples of the fluorescent substances include fluorescent dyes such as fluorescein isothiocyanate (FITC), rhodamine and Alexa Fluor (registered trademark), fluorescent proteins such as GFP, and the like. Examples of the radioisotopes include ¹²⁵I, ¹⁴C, ³²P, and the like. Among them, as the labeling substance, an enzyme is preferred, and alkaline phosphatase is particularly preferred.

The labeled anti-nucleophosmin antibody is obtained by labeling an anti-nucleophosmin antibody with the above labeling substance, according to a labeling method known in the art. An anti-nucleophosmin antibody may be labeled using a commercially available labeling kit or the like. The labeled immunoglobulin antibody may be obtained by using the same method as labeling of an anti-nucleophosmin antibody, or a commercially available product may be used.

In the present step, a signal generated by the labeling substance of the labeled anti-nucleophosmin antibody contained in the complex is detected, whereby a measured value of nucleophosmin contained in the biological sample can be obtained. The phrase "a signal is detected" includes qualitatively detecting the presence or absence of a signal, quantifying signal intensity, and semiquantitatively detecting signal intensity. The semiquantitative detection refers to indication of signal intensity in stages like "no signal generated", "weak", "middle", or "strong". In the present step, it is preferred that signal intensity is detected quantitatively or semiquantitatively.

The method itself for detecting a signal is known in the art. In the present step, a measurement method according to the kind of the signal derived from the above labeling substance may be properly selected. For example, when the labeling substance is an enzyme, a signal of light or color generated by reacting a substrate on the enzyme can be measured using a known device such as a luminometer or spectrophotometer.

The substrate of enzyme can be properly selected from known substrates according to the kind of the enzyme. When alkaline phosphatase is used as the enzyme, examples of the substrate include chemiluminescent substrates such as CDP-Star (registered trademark) (disodium 4-chloro-3-(methoxyspiro[1,2-dioxetane-3,2'-(5'-chloro)tricyclo[3.3.1.13,7]decan]-4-yl)phenyl phosphate) and CSPD (registered trademark) (disodium 3-(4-methoxyspiro[1,2-dioxetane-3,2-(5'-chloro)tricyclo[3.3.1.13,7]decan]-4-yl)phenyl phosphate), and chromogenic substrates such as 5-bromo-4-chloro-3-indolyl phosphate (BCIP), disodium 5-bromo-6-chloro-indolyl phosphate and p-nitrophenyl phosphoric acid. Particularly preferred is CDP-Star (registered trademark). Luminescence of the substrate is preferably detected by a luminometer.

When the labeling substance is a radioisotope, radiation as a signal can be measured using a known device such as a scintillation counter. When the labeling substance is a fluorescent substance, fluorescence as a signal can be measured using a known device such as a fluorescence microplate leader. Excitation wavelength and fluorescence wavelength can be properly determined according to the kind of the used fluorescent substance.

The detection result of signal can be used as the measured value of a nucleophosmin protein. For example, when the signal intensity is quantitatively detected, the measured value of signal intensity itself or the value calculated from the measured value of signal intensity can be used as the measured value of a nucleophosmin protein. Examples of the value calculated from the measured value of signal intensity include a value obtained by subtracting a measured value of signal intensity of a negative control sample from the measured value of signal intensity, a value obtained by dividing the measured value of signal intensity by a measured value of signal intensity of a positive control sample, a combination thereof, and the like. Examples of the negative control sample include a biological sample of a healthy subject, and the like. Examples of the positive control sample include a biological sample containing a nucleophosmin protein in a predetermined amount or concentration.

In order to obtain the measured value of a nucleophosmin protein, for example, a commercially available ELISA kit detecting a human nucleophosmin protein such as Human Nucleophosmin (NPM) ELISA Kit (catalogue number: MBS2024060) of MyBioSource, Inc. (San Diego, California, USA) can be also used. When the measured value of nucleophosmin in a biological sample is obtained using a commercially available kit, the measured value can be obtained according to the protocol attached to the kit.

### (i-2) Obtainment of expression level of nucleophosmin gene

When the measured value of nucleophosmin is an expression level of a nucleophosmin gene, a known method such as a microarray method, an RNA-seq analysis method or a quantitative RT-PCR method can be used for obtaining the expression level in the present step. As for a probe used in the microarray method, a probe selected by itself or a known probe may be synthesized and used, or a commercially available microarray chip may be used. Quantification by the microarray method can be carried out according to a known method, and the expression level of nucleophosmin mRNA may be expressed as an expression level relative to the expression level of housekeeping gene, and can be expressed as a measured value of signal intensity of a fluorescent dye or the like.

Quantification by RT-PCR can be carried out by performing a reverse transcription reaction using total RNA or mRNA extracted from a biological sample as a template, and analyzing by a realtime PCR method or the like using a specific primer of nucleophosmin mRNA using the obtained cDNA as a template. In this case, the expression level of nucleophosmin mRNA may be expressed as an expression level relative to the expression level of housekeeping gene, and may be expressed as a measured value of intensity of signal such as a fluorescent dye.

In the RNA-seq analysis method, the mRNA extracted from a biological sample is fragmented, and synthesis and library creation of cDNA are performed by a reverse transcription reaction using the fragmented mRNA as a template. As to the fragment contained in each library, a base sequence is determined by a next generation sequencer, and the information is mapped to a reference gene sequence, then the expression level of mRNA is expressed as RPKM (Reads Per Killobases per Million). RPKM may be expressed as intensity of signal such as heat map.

The detection result of the signal can be used as the expression level of nucleophosmin mRNA. For example, when the signal intensity is quantitatively detected, the measured value of signal intensity itself or the value calculated from the measured value of signal intensity can be used as the expression level of nucleophosmin mRNA.

Examples of the value calculated from the measured value of signal intensity include a value obtained by subtracting a measured value of signal intensity of a negative control sample from the measured value of signal intensity, a value obtained by dividing the measured value of signal intensity by a measured value of signal intensity of a positive control sample, a combination thereof, and the like. Examples of the negative control sample include a biological sample of a healthy subject, and the like. Examples of the positive control sample include a biological sample containing nucleophosmin mRNA in a predetermined expression level.

### (ii) Step of obtaining information on atrial fibrillation based on measured value of nucleophosmin

Next, information on atrial fibrillation is obtained based on the measured value of nucleophosmin obtained in the step (i) above. More specifically, the measured value of nucleophosmin in a biological sample of a test subject and the predetermined threshold are compared according to a known method such as a simple comparison method and a statistical test. Subsequently, when the measured value of nucleophosmin in a biological sample of a test subject is the threshold or more, it can be determined that the test subject has atrial fibrillation or is suspected to have atrial fibrillation. When the measured value of nucleophosmin in a biological sample of a test subject is lower than the threshold, it can be determined that the test subject does not have atrial fibrillation. The information on atrial fibrillation in a (test) subject is, for example, information on whether or not the test subject has atrial fibrillation, information on whether or not the test subject is suspected to have atrial fibrillation, and the like, obtained by the present step. Preferably, when the measured value of nucleophosmin in a biological sample of a test subject is the threshold or more, information showing that a test subject has atrial fibrillation is obtained. The obtained information is provided for supporting a doctor to diagnose that the test subject has atrial fibrillation or to diagnose that the test subject does not have atrial fibrillation.

The description concerning "test subject", "biological sample", "measured value of nucleophosmin", "predetermined threshold" and the like described in the "1. Explanation of terms" above may be incorporated herein.

### 3. Biomarker for determining atrial fibrillation

The present disclosure includes a biomarker for determining whether or not a test subject has atrial fibrillation, in the "2. Method for determining atrial fibrillation" described above. The biomarker contains a nucleophosmin protein present in a biological sample of an individual, preferably a peptide containing all or part of the amino acid sequence of SEQ ID NO: 1 or 2, and nucleophosmin mRNA, preferably a nucleic acid containing all or part of the base sequence of SEQ ID NO: 3 or 4. In the methods provided herein, the biomarker in a biological sample of a test subject is measured, and whether or not the test subject has atrial fibrillation can be determined based on the obtained measured value.

The description concerning "test subject", "biological sample", "measured value of nucleophosmin", "predetermined threshold" and the like described in the "1. Explanation of terms" above may be incorporated herein.

### 4. Inspection reagent for atrial fibrillation containing anti-nucleophosmin antibody

The present disclosure provides an inspection reagent for atrial fibrillation containing an anti-nucleophosmin antibody used in the "2. Method for determining atrial fibrillation" described above.

As the anti-nucleophosmin antibody, those described in the "1. Explanation of terms" described above can be used.

The inspection reagent may contain at least one or more anti-nucleophosmin antibodies. When the anti-nucleophosmin antibody is a polyclonal antibody, the anti-nucleophosmin antibody may be a polyclonal antibody obtained by immunization with one kind of antigen, or the anti-nucleophosmin antibody may be a polyclonal antibody obtained by immunizing the same individual with two or more kinds of antigens in parallel. Polyclonal antibodies each obtained by respectively inoculating two or more kinds of antigens into different animals may be mixed. When the anti-nucleophosmin antibody is a monoclonal antibody, the anti-nucleophosmin antibody may be a monoclonal antibody produced from one kind of hybridoma, or the anti-nucleophosmin antibody may be a monoclonal antibody produced from two or more kinds of hybridomas, and two or more kinds of a plurality of monoclonal antibodies in which each monoclonal antibody recognizes the same or different epitope may be contained. One or more kinds of polyclonal antibodies and one or more kinds of monoclonal antibodies may be mixed and contained.

The form of an anti-nucleophosmin antibody contained in the inspection reagent is not particularly limited. The form of an anti-nucleophosmin antibody contained in the inspection reagent may be a dry state or liquid state of antisera or ascites containing an anti-nucleophosmin antibody or the like. The form of an anti-nucleophosmin antibody may be a dry state or aqueous solution of a purified anti-nucleophosmin antibody, an immunoglobulin fraction containing an anti-nucleophosmin antibody or an IgG fraction containing an anti-nucleophosmin antibody.

When the form is a dry state or liquid state of antisera or ascites containing an anti-nucleophosmin antibody, at least one of stabilizers such as β-melcaptoethanol and DTT; protective agents such as albumin; surfactants such as polyoxyethylene(20) sorbitan monolaurate and polyoxyethylene(10) octylphenyl ether; preservatives such as sodium azide and the like may be further contained. When the form of an anti-nucleophosmin antibody is a dry state or an aqueous solution of a purified anti-nucleophosmin antibody, an immunoglobulin fraction containing an anti-nucleophosmin antibody or an IgG fraction containing an anti-nucleophosmin antibody, at least one of buffer components such as a phosphate buffer solution; stabilizers such as β-melcaptoethanol and DTT; protective agents such as albumin; salts such as sodium chloride; surfactants such as polyoxyethylene(20) sorbitan monolaurate and polyoxyethylene(10) octylphenyl ether; and preservatives such as sodium azide may be contained.

In the present disclosure, the anti-nucleophosmin antibody may be unlabeled, or may be labeled with biotin or the above-described labeling substance. The anti-nucleophosmin antibody is preferably labeled with biotin or the above-described labeling substance. As the labeling substance, those exemplified in the section of the "2. Method for determining atrial fibrillation" described above can be used, and alkaline phosphatase is preferably used. In the present disclosure, an anti-nucleophosmin antibody may be provided in a state immobilized on the solid phase surface or the like. The solid phase and immobilization are as exemplified in the section of the "2. Method for determining atrial fibrillation" described above. The solid phase is preferably a magnetic bead.

### 5. Inspection kit for atrial fibrillation

The present disclosure is an inspection kit for atrial fibrillation containing the "4. An inspection reagent for atrial fibrillation containing an anti-nucleophosmin antibody" described above, used in a method for detecting an atrial fibrillation patient or a method for identifying heart disease.

More specifically, the present disclosure is an inspection kit for atrial fibrillation containing an inspection reagent containing an anti-nucleophosmin antibody labeled with biotin and an inspection reagent containing an anti-nucleophosmin antibody labeled with a labeled substance. The labeling substance is preferably alkaline phosphatase. The kit may further contain a solid phase, preferably a solid phase to which avidins are bound, and more preferably a magnetic bead to which avidins are bound. The kit may contain a substrate. The substrate is preferably CDP-Star (registered trademark).

Details of the labeling substance, solid phase and substrate are as described in the "2. Method for determining atrial fibrillation", and the description may be incorporated herein.

The inspection kit preferably contains two kinds of anti-nucleophosmin antibodies bound to different epitopes on a nucleophosmin protein (first anti-nucleophosmin antibody, second anti-nucleophosmin antibody). In this case, a complex of a first anti-nucleophosmin antibody, nucleophosmin, a second anti-nucleophosmin antibody and a labeling substance is formed on the solid phase. The detection method as described above is generally called as sandwich ELISA. In this complex, the first anti-nucleophosmin antibody is immobilized on the solid phase, and the second anti-nucleophosmin antibody is directly or indirectly bound to the labeling substance. The phrase "the second anti-nucleophosmin antibody and the labeling substance are indirectly bound" refers to that the labeling substance is bound to the second anti-nucleophosmin antibody via an antibody or the like. Examples include a state that the labeling antibody recognizing the second anti-nucleophosmin antibody is bound to the second anti-nucleophosmin antibody.

When the first anti-nucleophosmin antibody is previously bound to the solid phase, the inspection kit can contain the solid phase on which the first anti-nucleophosmin antibody is immobilized, the second anti-nucleophosmin antibody, and the labeling substance. The second anti-nucleophosmin antibody and the labeling substance may be stored in separate containers, or may be stored in the same container. When the labeling substance is an enzyme and the inspection kit further contains a substrate, the enzyme and the substrate need to be stored in separate containers. When provided to a user, at least two kinds among the solid phase, the second anti-nucleophosmin antibody and the labeling substance may be packed together, or may be separately packed.

When the first anti-nucleophosmin antibody is not previously bound to the solid phase, the inspection kit can contain the solid phase, the first anti-nucleophosmin antibody, the second anti-nucleophosmin antibody, and the labeling substance. At least two of the first anti-nucleophosmin antibody, the second anti-nucleophosmin antibody and the labeling substance may be stored in the same container, or may be stored in separate containers. When the labeling substance is an enzyme and the inspection kit further contains a substrate, the enzyme and the substrate need to be stored in separate containers. When provided to a user, at least two of the solid phase, the first anti-nucleophosmin antibody, the second anti-nucleophosmin antibody and the labeling substance may be packed together, or may be separately packed.

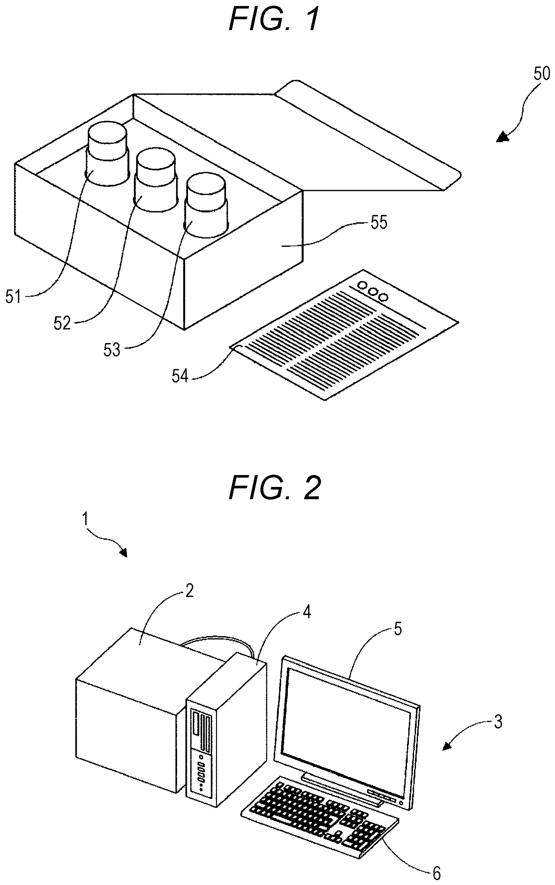

The above-described inspection kit can be provided to a user as a kit as shown in Fig. 1. An inspection kit 50 for atrial fibrillation according to this embodiment contains an outer packaging box 55, a first container 51 containing a plurality of magnetic particles as a solid phase, a second container 52 containing a first anti-nucleophosmin antibody which can be bound to the solid phase, a third container 53 containing an enzyme-labelled second anti-nucleophosmin antibody, and an attached document 54 of the inspection kit. In the attached document 54, a method for handling an inspection kit, storage conditions, an expiration date, and the like can be described. A container containing a substrate reagent, a container containing an aqueous medium for washing and the like may be packed together in the packaging box 55.

The present application also provides the use of an anti-nucleophosmin antibody, for producing an inspection reagent or inspection kit for atrial fibrillation. The inspection reagent, inspection kit and anti-nucleophosmin antibody are as described above.

### 6. Device for supporting diagnosis of atrial fibrillation, and program for supporting diagnosis of atrial fibrillation

Herein below, an embodiment of a diagnosis supporting device and diagnosis supporting program for performing the method described above will be described with reference to the attached drawings. Fig. 2 is a schematic view of a diagnosis supporting device 1. The diagnosis supporting device 1 contains a measurement device 2 and a computer system 3 connected to the measurement device 2.

The measurement device 2 measures a measured value of nucleophosmin in a biological sample collected from a test subject. The measurement device 2 is not particularly limited. The measurement device 2 can be properly selected depending on the method for measuring nucleophosmin. When the measured value of nucleophosmin is a measured value of a nucleophosmin protein, the measurement device 2 of the present embodiment is a measurement device capable of detecting a signal generated by ELISA method using a biotin-labeled anti-nucleophosmin antibody, magnetic particles on which avidins are immobilized, and an anti-nucleophosmin antibody labeled with a labeling substrate. This kind of measurement device is not particularly limited, as long as it is possible to detect a signal based on the used labeling substrate. The measurement device can be properly selected according to the kind of the labeling substrate.

When a biotin-labeled anti-nucleophosmin antibody, an inspection reagent containing magnetic particles on which avidins are immobilized, and a reagent containing an anti-nucleophosmin antibody labeled with a labeling substrate and a biological sample collected from a test subject are set in the measurement device 2, the measurement device 2 performs antigen-antibody reaction using each reagent. The measurement device 2 obtains a signal as an optical information based on a labeled antibody specifically bound to nucleophosmin. The measurement device 2 sends the obtained optical information to the computer system 3.

When the measured value of nucleophosmin is an expression level of a nucleophosmin gene, the measurement device 2 is a measurement device capable of detecting a signal generated by any of a microarray analysis, an RNA-seq analysis or a quantitative RT-PCR analysis. This kind of measurement device is not particularly limited, as long as it is possible to detect a signal based on the used labeling substrate. The measurement device can be properly selected according to the kind of the labeling substrate.

When mRNA extracted from a biological sample collected from a test subject or a reverse transcription product of the mRNA (may be labeled with a fluorescent dye or the like as necessary) is set in the measurement device 2 having a function to perform any processing of a microarray analysis, an RNA-seq analysis and a quantitative RT-PCR analysis, the measurement device 2 performs a predetermined processing. The measurement device 2 obtains a signal as an optical information based on the expression level of the nucleophosmin mRNA. The measurement device 2 sends the obtained optical information to the computer system 3.

The computer system 3 contains a computer 4, an input part 5 for inputting data and the like, and a display part 6 for displaying information of a test subject, a detection result and the like. The computer system 3 obtains a measured value of nucleophosmin in a biological sample collected from a test subject, based on the optical information received from the measurement device 2. The computer system 3 detects whether or not the test subject has atrial fibrillation, based on the measured value of nucleophosmin. The computer system 3 may be a separate equipment from the measurement device 2, as shown in Fig. 2, or the computer system 3 may be incorporated in the measurement device 2.

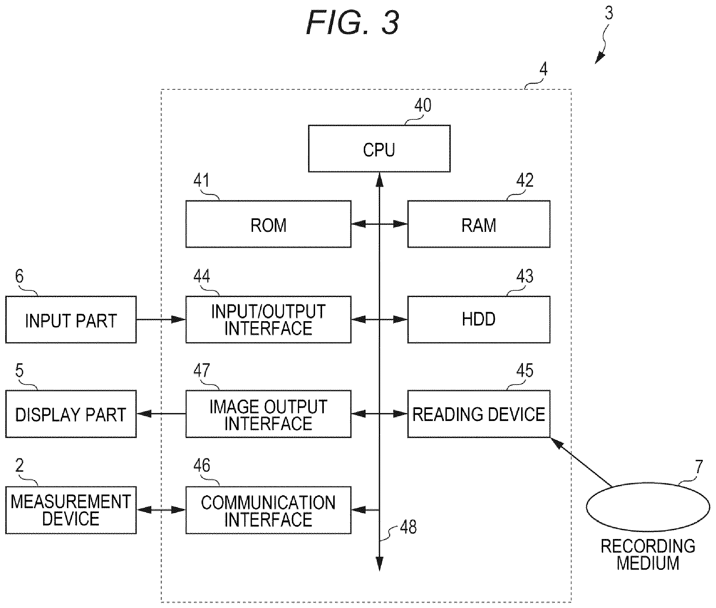

The computer 4 includes a processor (CPU) 40, an ROM 41, an RAM 42, a hard disk (HDD) 43, an input/output interface 44, a reading device 45, a communication interface 46 and an image output interface 47, as shown in Fig. 3, and these are connected via a bus 48 in a data-communicable manner. The measurement device 2 is communicably connected with the computer 4 by the communication interface 46.

The CPU 40 serially controls operation of each input and output part. The CPU 40 also executes a computer program stored in the ROM 41 or the hard disk 43. Namely, the CPU 40 processes the optical information received from the measurement device 2, in accordance with the computer program. The CPU 40 calculates the measured value of nucleophosmin in a biological sample. The CPU 40 also reads a predetermined threshold (cut off value) stored in the ROM 41 or the hard disk 43, and when the measured value of nucleophosmin is the predetermined threshold or more, the test subject collected from a biological sample is determined to have atrial fibrillation. Moreover, the CPU 40 outputs a determination result to display it on the display part 6.

The ROM 41 comprises a mask ROM, a PROM, an EPROM, an EEPROM, and the like. In the ROM 41, a computer program for detection of atrial fibrillation (program for supporting a diagnosis of atrial fibrillation) to be executed by the CPU 40 and a data used for executing the program for supporting a diagnosis of atrial fibrillation are recorded, as described above. In the ROM 41, in addition to the predetermined threshold, the past measured value of nucleophosmin of the test subject and the like may be recorded.

The RAM 42 comprises a SRAM, a DRAM, and the like. The RAM 42 is used for reading computer programs recorded in the ROM 41 and the hard disk 43. The RAM 42 is used as a working area of the CPU 40 when the CPU 40 executes these computer programs.

In the hard disk 43, an operation system and computer programs such as an application program (program for supporting a diagnosis of atrial fibrillation) for making the CPU 40 execute and a data used for executing the computer programs are recorded. In the hard disk 43, in addition to the predetermined threshold (cut off value), the past measured value of nucleophosmin of the test subject and the like may be recorded.

The reading device 45 comprises a flexible disk drive, a CD-ROM drive, a DVD-ROM drive, and the like. The reading device 45 can read a computer program or data recorded in a portable recording medium 7.

The input/output interface 44 comprises, for example, a serial interface such as USB, IEEE1394 or RS-232C, a parallel interface such as SCSI, IDE or IEEE1284, and an analogue interface comprising a D/A converter, an A/D converter or the like. To the input/output interface 44, the input part 5 such as a keyboard and a mouse is connected. An operator can input various commands to the computer 3 by the input part 5.

Examples of the communication interface 46 are Ethernet (registered trademark) interface and the like. The computer 4 can send print data to a printer or the like, through the communication interface 46.

The image output interface 47 is connected to the display part 6 comprising LCD, CRT and the like. The display part 6 can output a video signal corresponding to the image data given from the CPU 40. The display part 6 displays an image according to an input video signal.

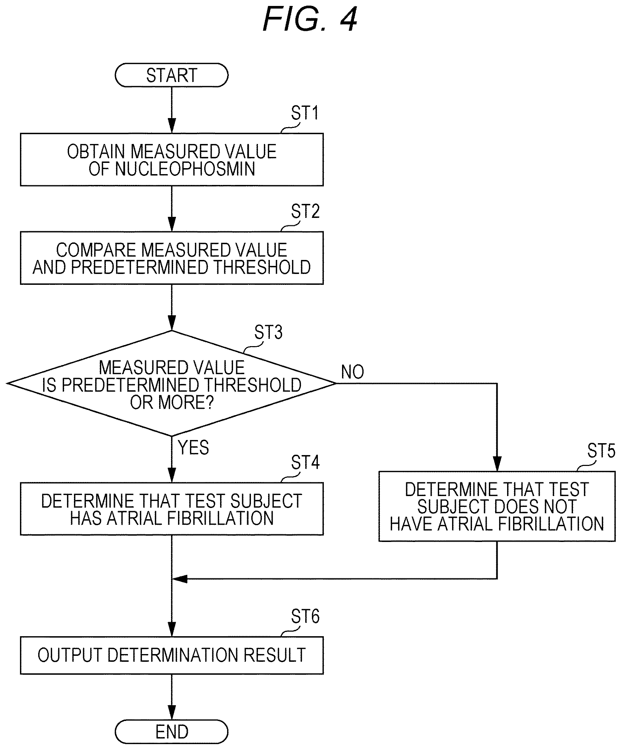

Next, a method for supporting a diagnosis of atrial fibrillation executed by the diagnosis supporting device 1 will be described, based on the program for supporting a diagnosis of atrial fibrillation, using Fig. 4. First, in Step ST1, when the CPU 40 receives transmission of optical information (signal) from the measurement device 2, the CPU 40 calculates the measured value of nucleophosmin in a biological sample from the obtained optical information. The CPU 40 stores the calculated measured value in the ROM 41 or the hard disk 43. Then, in Step ST2, the CPU 40 compares the obtained measured value of nucleophosmin and the predetermined threshold (cut off value) stored in the ROM 41 or the hard disk 43. When the measured value of nucleophosmin is the predetermined threshold (cut off value) or more, it is "YES" in Step ST3 and proceeds to ST4, then the CPU 40 determines that the test subject from which the biological sample is collected has atrial fibrillation, and in Step ST6, the CPU 40 outputs a determination result to display it on the display part 6, and print it out by a printer. The result that the test subject from which the biological sample is collected has atrial fibrillation may be stored in the ROM 41 or the hard disk 43.

On the other hand, when the measured value of nucleophosmin is lower than the predetermined threshold (cut off value), it is "NO" in Step ST3 and proceeds to ST5, and the CPU 40 determines that the test subject from which the biological sample is collected does not have atrial fibrillation. Then, in Step ST6, the CPU 40 outputs a determination result to display it on the display part 6, and print it out by a printer. The result that the test subject from which the biological sample is collected does not have atrial fibrillation may be stored in the ROM 41 or the hard disk 43.

Such information is provided to a doctor, as information for supporting to diagnose that the test subject has atrial fibrillation or to diagnose that the test subject does not have atrial fibrillation.

### EXAMPLES

Hereinbelow, the present invention will be further described in detail, with reference to Examples, but the present invention is not construed as being limited to the embodiments of the Examples.

### Examples: Correlation between measurement of nucleophosmin concentration in serum and various heart diseases

In order to compare the nucleophosmin concentration in the serum of each of heart disease patients, the nucleophosmin concentration in the serum of each test subject in six groups shown in Table 1 was measured.

### 1. Subjects

The details of test subjects are shown in Table 1.

**[Table 1]**

| Test subject | Number of people |
|---|---|
| Atrial fibrillation (Af) patient group | 10 |
| Dilated cardiomyopathy (DCM) patient group | 15 |
| Hypertrophic cardiomyopathy (HCM) patient group | 10 |
| Hypertension (HT) patient group | 10 |
| Ischemic heart failure (IHF) patient group | 15 |
| Healthy subject group | 10 |

A healthy subject is a person who is not suspected of a disease including not only a heart disease, but also other diseases.

### 2. Measurement method

Blood was collected from the test subject, and the serum was separated. Thereafter, the serum was frozen and stored at -80°C until measurement.

Nucleophosmin in the serum was measured according to the attached protocol, using Human Nucleophosmin (NPM) ELISA Kit (catalogue number: MBS2024060) purchased from MyBioSource, Inc. (San Diego, California, USA).

StatFlex was used for statistical analysis, and fundamental statistic (parametric method), one-way analysis of variance and independent multigroup multiple comparison (Tukey's test) were performed. ROC analysis was performed for tests of sensitivity and specificity, and the AUC value was further obtained.

### 3. Result (1) Nucleophosmin concentration in serum

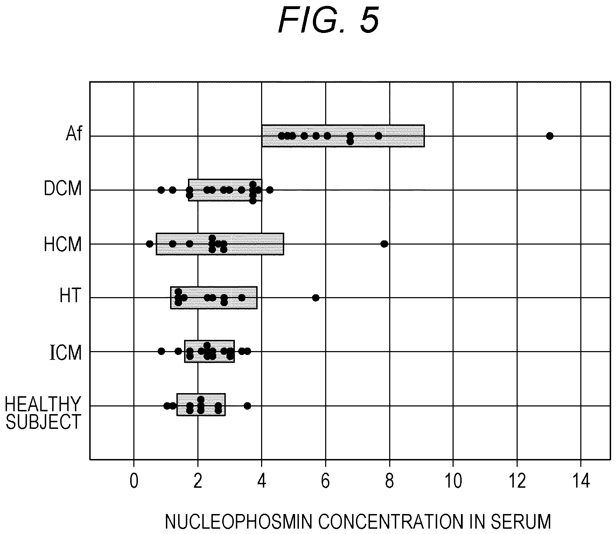

Average ± standard deviation (ng/ml) of the nucleophosmin concentration in the serum of each group obtained by a parametric method is shown in Table 2. A scatter diagram of a nucleophosmin concentration in the serum of each group is shown in Fig. 5.

**[Table 2]**

| | Nucleophosmin concentration in serum (ng/ml) |
|---|---|
| Af patient group | 6.58±2.48 |
| DCM patient group | 2.86±1.09 |
| HCM patient group | 2.71±1.94 |
| HT patient group | 2.54±1.31 |
| ICM patient group | 2.37±0.73 |
| Healthy subject group | 2.11±0.71 |

The results of Tukey's test are shown in Table 3. As shown in Table 3, q values of the Af patient group against other groups all showed P<0.01, thus it was shown that the measured value of nucleophosmin in the serum was significantly increased in the Af patient group as compared to other disease groups.

**[Table 3]**

| | | Af patient group | DCM patient group | HCM patient group | HT patient group | ICM patient group | Healthy subject group |
|---|---|---|---|---|---|---|---|
| Af patient group | q value | | -7.9469 | -7.5333 | -7.8803 | -8.9785 | -8.7145 |
| | Significance | | P < 0.01 | P < 0.01 | P < 0.01 | P < 0.01 | P< 0.01 |
| DCM patient group | q value | 7.9469 | | -0.3055 | -0.6855 | -1.1533 | -1.5994 |
| | Significance | P < 0.01 | | NS | NS | NS | NS |
| HCM patient group | q value | 7.5333 | 0.3055 | | -0.3470 | -0.7261 | -1.1812 |
| | Significance | P < 0.01 | NS | | NS | NS | NS |
| HT patient group | q value | 7.8803 | 0.6855 | 0.3470 | | -0.3460 | -0.8342 |
| | Significance | P < 0.01 | NS | NS | | NS | NS |
| ICM patient group | q value | 8.9785 | 1.1533 | 0.7261 | 0.3460 | | -0.5678 |
| | Significance | P < 0.01 | NS | NS | NS | | NS |
| Healthy subject group | q value | 8.7145 | 1.5994 | 1.1812 | 0.8342 | 0.5678 | |
| | Significance | P < 0.01 | NS | NS | NS | NS | |

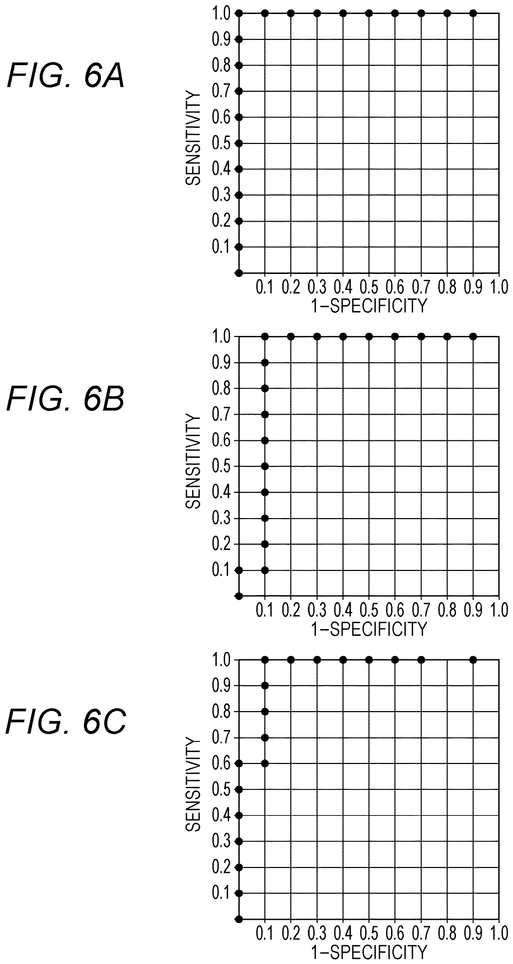

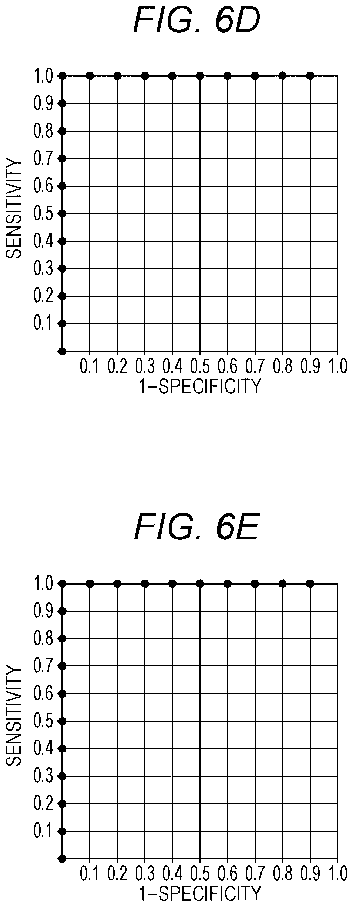

Next, ROC analysis was performed to study the sensitivity and specificity of the measured value of nucleophosmin in the prediction or diagnosis of atrial fibrillation. ROC curves of the measured value of nucleophosmin of the Af patient group for the measured values of nucleophosmin of the healthy subject group and each of the heart disease patient groups are shown in Fig. 6A-E. The ROC curves had a shape along the upper left corner. AUC values for the Af patient group against each of the disease groups and AUC values of the healthy subject group against each of the disease groups are shown in Table 4. The AUC values for the Af patient group against each of the disease groups were 0.91 to 1.00 that were classified into high accuracy (Table 4). On the other hand, the AUC values for the healthy subject group against each of the disease groups were 0.53 to 0.72 that were classified into low accuracy (Table 4). Based on the above, it became clear that the measured value of nucleophosmin is excellent in predictive ability or diagnostic ability of atrial fibrillation.

**[Table 4]**

| | Af patient group | Healthy subject group |
|---|---|---|
| Against DCM patient group | 1.00 | 0.72 |
| Against HCM patient group | 0.91 | 0.62 |
| Against HT patient group | 0.96 | 0.53 |
| Against ICM patient group | 1.00 | 0.63 |
| Against healthy subject group | 1.00 | |

In the present example, information on atrial fibrillation was obtained using the measured value of a nucleophosmin protein as an index. Since the expression level of a nucleophosmin protein and the expression level of a nucleophosmin gene have a strong correlation, it is considered that information on atrial fibrillation can be obtained also using the expression level of a nucleophosmin gene as an index.

## Claims

1. A method for supporting a diagnosis of atrial fibrillation comprising:
(a) a step of obtaining a measured value of nucleophosmin in a biological sample collected from a test subject; and
(b) a step of obtaining information on atrial fibrillation comprising comparing the measured value of nucleophosmin and a predetermined threshold,
wherein information showing that the test subject has atrial fibrillation or is suspected to have atrial fibrillation is obtained when the measured value is the threshold or more, or
wherein information showing that the test subject does not have atrial fibrillation is obtained when the measured value is less than the threshold.

2. The method according to claim 1, wherein the biological sample is a blood sample.

3. The method according to any one of claims 1 or 2, wherein the measured value of nucleophosmin is an expression level of a nucleophosmin gene or a measured value of a nucleophosmin protein.

4. *In vitro* use of a reagent for the method for supporting a diagnosis of atrial fibrillation according to any one of claims 1 to 3, wherein
the reagent comprises an anti-nucleophosmin antibody.

5. *In vitro* use of a kit for the method for supporting a diagnosis of atrial fibrillation according to any one of claims 1 to 3, the kit comprising:
a first anti-nucleophosmin antibody;
a solid phase;
a second anti-nucleophosmin antibody; and
a labeling substance.

## Patentansprüche

1. Verfahren zum Unterstützen einer Diagnose von Vorhofflimmern, umfassend:
(a) einen Schritt des Erhaltens eines Messwerts für Nucleophosmin in einer biologischen Probe, die einer Testperson entnommen wurde, und
(b) einen Schritt des Erhaltens von Informationen zu Vorhofflimmern, umfassend das Vergleichen des Messwerts für Nucleophosmin und eines zuvor festgelegten Schwellenwerts,
wobei Informationen, die zeigen, dass die Testperson Vorhofflimmern hat oder der Verdacht besteht, dass sie Vorhofflimmern hat, erhalten werden, wenn der Messwert der Schwellenwert oder größer ist, oder
wobei Informationen, die zeigen, dass die Testperson kein Vorhofflimmern hat, erhalten werden, wenn der Messwert kleiner als der Schwellenwert ist.

2. Verfahren nach Anspruch 1, wobei die biologische Probe eine Blutprobe ist.

3. Verfahren nach einem der Ansprüche 1 oder 2, wobei der Messwert für Nucleophosmin ein Expressionsspiegel eines Nucleophosmin-Gens oder ein Messwert eines Nucleophosmin-Proteins ist.

4. In vitro-Verwendung eines Reagenzes für das Verfahren zum Unterstützen einer Diagnose von Vorhofflimmern nach einem der Ansprüche 1 bis 3, wobei
das Reagenz einen Anti-Nucleophosmin-Antikörper umfasst.

5. In *vitro*-Verwendung eines Kits für das Verfahren zum Unterstützen einer Diagnose von Vorhofflimmern nach einem der Ansprüche 1 bis 3, wobei das Kit Folgendes umfasst:
einen ersten Anti-Nucleophosmin-Antikörper,
eine feste Phase,
einen zweiten Anti-Nucleophosmin-Antikörper und
eine Markierungssubstanz.

## Revendications

1. Procédé de support d'un diagnostic de fibrillation atriale comprenant :
(a) une étape consistant à obtenir une valeur mesurée de nucléophosmine dans un échantillon biologique prélevé à partir d'un sujet d'essai ; et
(b) une étape consistant à obtenir des informations sur la fibrillation atriale comprenant la comparaison de la valeur mesurée de nucléophosmine et d'un seuil prédéterminé,
des informations indiquant que le sujet d'essai est atteint de fibrillation atriale ou est soupçonné d'être atteint de fibrillation atriale étant obtenues lorsque la valeur mesurée est supérieure ou égale au seuil, ou
des informations indiquant que le sujet d'essai n'est pas atteint de fibrillation atriale étant obtenues lorsque la valeur mesurée est inférieure au seuil.

2. Procédé selon la revendication 1, dans lequel l'échantillon biologique est un échantillon de sang.

3. Procédé selon l'une quelconque des revendications 1 et 2, dans lequel la valeur mesurée de nucléophosmine est un niveau d'expression d'un gène de nucléophosmine ou une valeur mesurée d'une protéine nucléophosmine.

4. Utilisation *in vitro* d'un réactif pour le procédé de support d'un diagnostic de fibrillation atriale selon l'une quelconque des revendications 1 à 3, dans laquelle
le réactif comprend un anticorps anti-nucléophosmine.

5. Utilisation *in vitro* d'un kit pour le procédé de support d'un diagnostic de fibrillation atriale selon l'une quelconque des revendications 1 à 3, le kit comprenant :
un premier anticorps anti-nucléophosmine ;
une phase solide ;
un second anticorps anti-nucléophosmine ; et
une substance de marquage.
